# EUROPEAN PATENT APPLICATION

(11) **EP 4 368 653 A1**
(43) Date of publication of application: **15.05.2024**
(21) Application number: 22837533.3
(22) Date of filing: 28.06.2022
(51) Int. Cl.: C08G 65/332, C07C 69/602, C08F 2/46, C08F 290/06, C09J 4/02, C09J 175/14

(54) **DI(METH)ACRYLATE, PHOTOCURABLE RESIN COMPOSITION, AND PHOTOCURABLE RESIN COMPOSITION FOR ADHESIVE**

(30) Priority: 05.07.2021 JP 2021111779; 30.07.2021 JP 2021126067
(71) Applicant: NOF Corporation, Shibuya-ku Tokyo 150-6019 (JP)
(72) Inventor: NAGASAWA, Atsushi, Amagasaki-shi, Hyogo 660-0095 (JP); YAMADA, Ayaka, Amagasaki-shi, Hyogo 660-0095 (JP)
(74) Representative: Weickmann & Weickmann PartmbB
(86) International application number: PCT/JP2022/025666
(87) International publication number: WO 2023/282117

(57) **Abstract**

Provided are photocurable resin compositions having low viscosity before curing, superior in storage at low temperatures, and affording after curing a film superior in water resistance, bending resistance, and transparency, and photocurable resin compositions for adhesive superior in coatability onto concave-convex surfaces, and capable of forming a cured product (adhesive layer) superior in transparency, adhesiveness to uneven substrates, and flexibility during bending, and having high water-resistant adhesiveness. A di(meth)acrylate represented by the following formula (1): 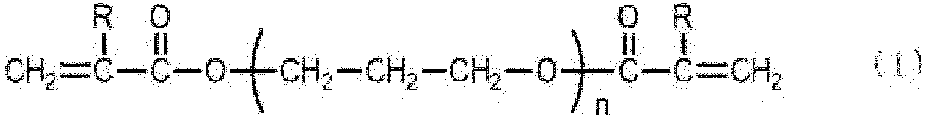 wherein R is a hydrogen atom or a methyl group, and n is an average number of moles of added oxytrimethylene group and is a real number of 5 to 90; a photocurable resin composition containing 30 to 100% by mass of component (A): di(meth)acrylate (A), and 0 to 70% by mass of component (B1): urethane (meth)acrylate (B1), with respect to a total of the monomer components (100% by mass) of the composition; and a photocurable resin composition for adhesive, including component (A): di(meth)acrylate (A); component (B): polymerizable oligomer (B) having two or more (meth)acryloyl groups in one molecule; and component (C1): monofunctional (meth)acrylate monomer (C1), wherein a content of component (A) is 1 to 50% by mass, a content of component (B) is 15 to 98% by mass, and a content of component (C1) is 1 to 35% by mass, with respect to a total (100% by mass) of component (A), component (B), and component (C1).

## Description

### [Technical Field]

The present invention relates to a di(meth)acrylate having a polytrimethylene glycol structure, and a photocurable resin composition and a photocurable resin composition for adhesive, each containing the di(meth)acrylate.

### [Background Art]

A photocurable resin composition containing a polyfunctional monomer or a monofunctional monomer having a (meth)acryloyl group is used for UV ink to be cured with a light source such as a UV lamp after being discharged, a hard coat film for protecting the surface of a substrate, and a resist film such as a dry film resist. In these applications, a method of photocuring using a polyfunctional monomer generally having two or more (meth)acryloyl groups is adopted. In recent years, applications requiring greater flexibility, such as flexible devices and coating on curved surfaces, have been increasing.

As a compound superior in flexibility, Patent Literature 1 describes a composition of a polyurethane acrylate having a urethane bond in the molecule and an alicyclic acrylate. Even though the obtained cured product is superior in flexibility, the composition shows poor coating property due to the high viscosity of the composition before curing. When the composition is mixed with a monofunctional monomer for dilution in order to lower the viscosity, physical properties such as flexibility and strength of the resulting cured product problematically decrease. Examples of the structure of a polyfunctional monomer with relatively low viscosity include a structure in which two (meth)acryloyl groups are linked with polyalkylene glycol. As the polyalkylene glycol, a polyethylene glycol structure, a poly 1,2-propylene glycol structure, a polytetramethylene glycol structure, and the like are generally used. However, although polyethylene glycol di(meth)acrylate has excellent hardness, it is highly water-soluble and causes problems of dissolution of the film and swelling due to moisture absorption.

Patent Literature 2 discloses a poly 1,2-propylene glycol di(meth)acrylate composition and a polytetramethylene glycol di(meth)acrylate composition that can form a coating film superior in impact resistance. However, since poly 1,2-propylene glycol di(meth)acrylate is relatively hard and brittle, it causes a problem of cracks and fissures that occur easily when used for applications requiring greater flexibility. In this respect, even though polytetramethylene glycol di(meth)acrylate characteristically has appropriate flexibility, since it has high crystallinity and high melting point, problems of solidification when stored at low temperatures or cloudy white coating film obtained after curing occur.

In addition, UV-curable adhesives do not require curing at high temperatures and permit adhesion in a short time, and therefore, they are used in various fields such as information·electronics and machinery industries. In the field of information·electronics, input devices with touch panel functions provided on the surface of liquid crystal display, organic EL display, and the like have recently been widely used in image display devices used in smartphones, and the like, and adhesives are also used in the manufacturing process thereof.

Touch panels generally have a structure in which multiple members such as a substrate, a transparent conductive film, and a design film are laminated, and adhesives have conventionally been used to bond these materials. However, there was a problem of easy entry of air bubbles and foreign matters during bonding, and a method of bonding using an ultraviolet curable adhesive is known as a technique in place of adhesives (Patent Literature 3).

In recent years, applications for flexible devices and adhesion of concave-convex surfaces such as design films and wiring printed circuit boards have increased, and coatability and adhesiveness onto concave-convex surfaces are required in addition to flexibility during bending. On the other hand, adhesives with high flexibility are easily affected by moisture, resulting in problems such as whitening of the formed cured product (adhesive layer) and a decrease in water-resistant adhesiveness.

Patent Literature 4 discloses an adhesive composition containing a (meth)acrylic polymer and polypropylene glycol di(meth)acrylate or polytetramethylene glycol di(meth)acrylate. Since polypropylene glycol di(meth)acrylate is relatively hard and brittle, it causes a problem of breakage and cracks that occur easily when used for applications requiring flexibility during bending. Even though polytetramethylene glycol di(meth)acrylate characteristically has appropriate flexibility, it has a high melting point and has problems with transparency.

Patent Literature 5 discloses an adhesive composition containing urethane (meth)acrylate, a hydroxyl group-containing monofunctional monomer, and polyester diol as a non-radically polymerizable flexible component. Since it contains a large amount of monofunctional monomer as a hydrophilic component, it has excellent elongation (also to be referred to as flexibility). However, since the crosslinking density decreases, sufficient adhesiveness is difficult to obtain, and there was a risk that the water-resistant adhesiveness of the cured product formed (adhesive layer) would decrease due to moisture absorption and swelling under high humidity.

### [Citation List]

### [Patent Literature]

[Patent Literature 1]
   JP-A-2019-086783
[Patent Literature 2]
   JP-A-2018-024764
[Patent Literature 3]
   JP-A-2004-77887
[Patent Literature 4]
   JP-A-2016-166360
[Patent Literature 5]
   JP-A-2014-118508

### [Summary of Invention]

### [Technical Problem]

In order to solve the above-mentioned problems, a photocurable resin composition has been desired that has low viscosity before curing, does not solidify during storage at low temperatures, affords after curing a film with high water resistance, is superior in flexibility, and does not become cloudy.

In addition, in order to solve the above-mentioned problems, an adhesive composition has been desired that is superior in coatability onto concave-convex surfaces, and capable of forming a cured product (adhesive layer) superior in transparency, adhesiveness to uneven substrates, and flexibility during bending, and having high water-resistant adhesiveness.

### [Solution to Problem]

The present inventors have conducted intensive studies in an attempt to solve the above-mentioned problems and found that a photocurable resin composition and a photocurable resin composition for adhesive, each containing di(meth)acrylate having a polytrimethylene glycol structure, can solve the above-mentioned problems.

Accordingly, the present invention provides the following.
[1] A di(meth)acrylate represented by the following formula (1) : 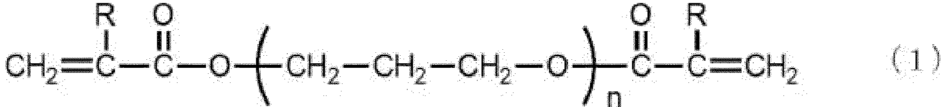 wherein R is a hydrogen atom or a methyl group, and n is an average number of moles of added oxytrimethylene group and is a real number of 5 to 90.
[2] A photocurable resin composition comprising 30 to 100% by mass of component (A): di(meth)acrylate (A) in [1], and 0 to 70% by mass of component (B1): urethane (meth)acrylate (B1), with respect to the total of the monomer components (100% by mass) of the composition.
[3] A photocurable resin composition for adhesive, comprising
   component (A): di(meth)acrylate (A) in [1];
   component (B): polymerizable oligomer (B) having two or more (meth)acryloyl groups in one molecule; and
   component (C1): monofunctional (meth)acrylate monomer (C1), wherein
   a content of component (A) is 1 to 50% by mass, a content of component (B) is 15 to 98% by mass, and a content of component (C1) is 1 to 35% by mass, with respect to a total (100% by mass) of component (A), component (B), and component (C1) .
[4] The photocurable resin composition for adhesive of [3], wherein the aforementioned polymerizable oligomer (B) having two or more (meth)acryloyl groups in one molecule is a urethane (meth)acrylate oligomer having a polyether skeleton or a polyester skeleton.
[5] A cured product obtained by curing the photocurable resin composition for adhesive of [3] or [4].

### [Advantageous Effects of Invention]

The photocurable resin composition of the present invention has low viscosity before curing, is superior in storage at low temperatures, and affords after curing a film superior in water resistance, bending resistance, and transparency.

In addition, the photocurable resin composition for adhesive of the present invention is superior in coatability onto concave-convex surfaces, and capable of forming a cured product (adhesive layer) superior in transparency, adhesiveness to uneven substrates, and flexibility during bending, and having high water-resistant adhesiveness.

### [Description of Embodiments]

The embodiments of the present invention are described below, to which the present invention is not limited.

In the present specification, (meth)acrylate means acrylate or methacrylate. The (meth)acryloyl group means an acryloyl group or a methacryloyl group.

### [Photocurable resin composition]

The photocurable resin composition of the present invention is characterized in that it contains 30 to 100% by mass of component (A): di(meth)acrylate (A) represented by the formula (1), and 0 to 70% by mass of component (B1): urethane (meth)acrylate (B1), with respect to the total of the monomer components (100% by mass) of the composition.

### [di(meth)acrylate (A) (component (A))]

The di(meth)acrylate (A) in the present invention is represented by the following formula (1), i.e., polytrimethylene glycol di(meth)acrylate.

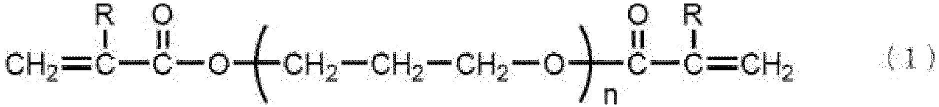

In the formula, R is a hydrogen atom or a methyl group.

In the formula, n is an average number of moles of added oxytrimethylene group and is a real number of 5 to 90. From the aspects of the viscosity of the resin composition before curing and water resistance of a film obtained after curing, n is preferably a real number of 7 to 50, more preferably a real number of 8 to 40.

One kind of di(meth)acrylate (A) may be used alone, or two or more kinds thereof may be used in combination.

From the aspect of bending resistance, di(meth)acrylate (A) preferably has an acid value of not more than 5.0 mgKOH/g, more preferably not more than 3.0 mgKOH/g. As used herein, the "acid value" means the number of mg of potassium hydroxide required to neutralize 1 g of a sample.

From the aspect of water resistance, di(meth)acrylate (A) preferably has a hydroxyl value of not more than 10.0 mgKOH/g, more preferably not more than 5.0 mgKOH/g. As used herein, the "hydroxyl value" means the number of milligrams of potassium hydroxide required to neutralize a sample obtained by treating a specimen by adding a mixture of acetic anhydride and pyridine, adding a small amount of water, and then heat-treating the mixture.

Furthermore, from the aspect of photocurability, di(meth)acrylate (A) preferably has a saponification value of 20 to 280 mgKOH/g, more preferably 40 to 200 mgKOH/g. As used herein, the "saponification value" means the number of milligrams of potassium hydroxide required to saponify 1 g of a sample.

The acid value, hydroxyl value, and saponification value can be measured by the methods defined in JIS K 0070:1992.

The amount of di(meth)acrylate (A) contained in the photocurable resin composition of the present invention is 30 to 100% by mass, preferably 50 to 90% by mass, more preferably 60 to 80% by mass, with respect to the total of the monomer components (100% by mass) of the composition, from the aspects of the viscosity of the resin composition before curing and the flexibility of the film obtained after curing.

### [Production method of di(meth)acrylate (A)]

Di(meth)acrylate (A) in the present invention is polytrimethylene glycol di(meth)acrylate as represented by the formula (1).

The above-mentioned di(meth)acrylate (A) can be obtained by, for example, an esterification reaction of polytrimethylene glycol with (meth)acrylic acid, a transesterification reaction with (meth)acrylic acid derivative, and the like.

As the (meth)acrylic acid, a derivative thereof may also be used. For example, a transesterification reaction may be performed using an ester of (meth)acrylic acid. As the ester, methyl ester, ethyl ester, propyl ester, butyl ester, and the like can be used. In addition, (meth)acrylic anhydride, (meth)acrylic halide, and the like may also be used. As the acid halide, acid chloride or acid bromide can be used. Furthermore, (meth)acrylic acid may be converted into an active ester with p-nitrophenyl ester, N-hydroxysuccinimide ester, pentafluorophenyl ester, or the like.

Di(meth)acrylate (A) can be easily obtained by esterification by reacting (meth)acrylic acid and polytrimethylene glycol in the presence of a catalyst.

Since (meth)acrylic acid is generally liquid at room temperature, the reaction can also be performed without solvent. Without solvent, however, the reaction may proceed excessively and the yield may decrease. Thus, it is preferable to use a solvent. The solvent is not particularly limited as long as the reaction is not inhibited. Examples of the usable solvent include aromatic hydrocarbon solvents such as toluene, xylene, and the like, hydrocarbon solvents such as normal hexane, cyclohexane, methylcyclohexane, and the like; halogenated hydrocarbon solvents such as dichloromethane, chloroform, and the like; ether solvents such as diethyl ether, tetrahydrofuran, and the like; ketone solvents such as acetone, ethylmethylketone, 4-methyl-2-pentanone, and the like; sulfoxide solvents such as dimethyl sulfoxide and the like, and the like. Ester solvents such as ethyl acetate can also be used except when performing transesterification reactions.

The reaction between (meth)acrylic acid and polytrimethylene glycol is a general esterification reaction, and may be performed by dehydration condensation in the presence of an esterification catalyst. As the esterification catalyst, for example, strong acids such as sulfuric acid, hydrogen bromide, hydrogen fluoride, boron trifluoride, p-toluenesulfonic acid, methanesulfonic acid, trifluoromethanoic acid, and the like, ion exchange resin, titanate ester, and the like can be recited as examples.

The reaction using (meth)acrylate ester is a general transesterification reaction, and heating is performed in the presence of a catalyst. As the catalyst, for example, sodium alkoxide, titanate ester, dibutyltin oxide, and the like can be recited as examples.

When an acid halide or an acid anhydride of (meth)acrylic acid is used, a base is used as a catalyst or for neutralization of acid. As the base, alkali metal hydroxides such as sodium hydroxide, potassium hydroxide, and the like; alkali metal carbonates such as potassium carbonate, sodium carbonate, and the like; alkali metal hydrogen carbonates such as potassium hydrogen carbonate, sodium hydrogen carbonate, and the like; organic amines such as pyridine, triethylamine, N,N-dimethyl-4-aminopyridine, and the like can be used.

These reactions are preferably performed in the presence of a polymerization inhibitor. Examples of the polymerization inhibitor include phenol compounds such as hydroquione, methylhydroquione, tert-butylhydroquione, 2,6-di-tert-butylhydroquione, 2,5-di-tert-butylhydroquione, paramethoxyphenol, 2,4-dimethyl-6-tert-butylphenol, hydroquione monomethyl ether, cresol, catechol, tert-butylcatechol, 2,6-di-tert-butyl-4-methylphenol, and the like; paraphenylenediamines such as paraphenylenediamine, N-isopropyl-N'-phenylparaphenylenediamine, N-(1,3-dimethylbutyl)-N'-phenylparaphenylenediamine, N-(1-methylheptyl)-N'-phenylparaphenylenediamine, N,N'-diphenyl-paraphenylenediamine, N,N'di-2-naphthyl-paraphenylenediamine, and the like; amine compounds such as thiodiphenylamine, phenothiazine, and the like; quiones such as benzoquinone, diphenylbenzoquinone, and the like; copper dialkyldithiocarbamates such as copper dibutyldithiocarbamate, copper dipropyldithiocarbamate, copper diethyldithiocarbamate, copper dimethyldithiocarbamate, and the like; copper diaryldithiocarbamates such as copper diphenyldithiocarbamate, and the like; nitroso compounds such as nitrosophenol, N-nitrosodiphenylamine, isoamyl nitrite, N-nitroso-cyclohexylhydroxylamine, N-nitroso-N-phenyl-N-hydroxylamine, and salts thereof, and the like; N-oxyl compounds such as 2,2,4,4-tetramethylazetidine-1-oxyl, 2,2-dimethyl-4,4-dipropylazetidine-1-oxyl, 2,2,5,5-tetramethylpyrrolidine-1-oxyl, 2,2,5,5-tetramethyl-3-oxopyrrolidine-1-oxyl, 2,2,6,6-tetramethylpiperidine-1-oxyl, 4-hydroxy-2,2,6,6-tetramethylpiperidine-1-oxyl, 6-aza-7,7-dimethyl-spiro(4,5)decane-6-oxyl, 2,2,6,6-tetramethyl-4-acetoxypiperidine-1-oxyl, 2,2,6,6-tetramethyl-4-benzoyloxypiperidine-1-oxyl, 4,4',4"-tris-(2,2,6,6-tetramethylpiperidine-1-oxyl)phosphite, and the like; tetraalkylthiuram disulfides such as tetrabutylthiuram disulfide, tetrapropylthiuram disulfide, tetraethylthiuram disulfide, tetramethylthiuram disulfide, and the like; methylene blue, and the like. Only one kind of these polymerization inhibitors may be used alone or two or more kinds thereof may be used in combination.

The water or lower alcohol produced by these reactions is preferably taken out of the system. These reactions can be performed in the presence of a suitable azeotropic solvent, such as hexane, toluene, xylene, and the like, where necessary.

The reaction temperature and reaction time may be determined as appropriate. For example, the reaction can be accelerated by increasing the reaction temperature, but when it is too high, the polymerization reaction may also proceed. Specifically, the temperature can be set to not less than about 40°C and not more than about 120°C. Depending on the solvent, heating may be performed under reflux, or a Dean-Stark apparatus may also be used. Regarding the reaction time, the progress of the reaction may be confirmed by, for example, thin layer chromatography or gas chromatography. Specifically, it can be not less than about 1 hour and not more than about 20 hours. More specifically, it can be determined by preliminary experiments or the like.

After completion of the reaction, a general posttreatment may be performed. For example, a water-immiscible organic solvent such as toluene or ethyl acetate is added to the reaction solution of di(meth)acrylate (A), and the mixture is washed with water to remove water-soluble components such as catalysts. After purification, the solvent is removed. Further, it may be purified by general purification means such as column chromatography.

### [Urethane (meth)acrylate (B1) (component (B1))]

The photocurable resin composition of the present invention may contain urethane (meth)acrylate (B1). The urethane (meth)acrylate (B1) preferably has 2 to 3 (meth)acryloyl groups in one molecule and has a weight average molecular weight of 5,000 to 50,000. Urethane (meth)acrylate means a compound having a urethane bond and a (meth)acryloyl group in one molecule.

When the number of (meth)acryloyl groups in one molecule of urethane (meth)acrylate (B1) is 2 to 3, the flexibility of the film obtained after curing the photocurable resin composition is easily improved. From such aspect, the number of (meth)acryloyl groups in one molecule of urethane (meth)acrylate (B1) is more preferably two.

The weight average molecular weight of urethane (meth)acrylate (B1) is preferably 5000 to 50000. When the weight average molecular weight is less than 5000, the flexibility of the film obtained after curing the photocurable resin composition becomes insufficient, and when the weight average molecular weight exceeds 50000, the viscosity of the photocurable resin composition becomes high and the handling property decreases.

From the aspect of improving the viscosity of the photocurable resin composition and the flexibility of the film obtained after curing the composition, the weight average molecular weight of the urethane (meth)acrylate (B1) is more preferably 7000 to 20000, further preferably 10000 to 15000. The weight average molecular weight can be determined based on polystyrene by using, for example, gel permeation chromatography (GPC).

The viscosity of urethane (meth)acrylate (B1) at 60°C is preferably 3000 to 70000 mPa·s, more preferably 4000 to 50000 mPa·s, further preferably 5000 to 30000 mPa·s, from the aspects of the handling property of the photocurable resin composition. The viscosity can be measured using, for example, E type viscosimeter.

The glass transition temperature of the cured product obtained by curing urethane (meth)acrylate (B1) alone is preferably not more than 20°C, more preferably not more than 10°C, further preferably not more than 0°C, from the aspect of obtaining sufficient flexibility at room temperature of the film obtained after curing the photocurable resin composition. The glass transition temperature can be measured using, for example, a differential scanning calorimeter.

The method for producing urethane (meth)acrylate (B1) is not particularly limited. For example, it can be obtained by reacting a polyhydric alcohol, a polyisocyanate such as isocyanate or isocyanurate, and a (meth)acrylate having a hydroxyl group or a (meth)acrylate having an isocyanate group. Example of the above-mentioned polyhydric alcohol include polyhydric alcohols having a polyether skeleton, polyester skeleton, or polycarbonate skeleton in the molecule. Among these, polyhydric alcohols having a polyalkylene glycol skeleton having 2 to 4 carbon atoms are preferred, and polyhydric alcohols having a polypropylene glycol skeleton are more preferred. Examples of the above-mentioned polyisocyanate include diisocyanates such as isophorone diisocyanate, 2,4-tolylene diisocyanate, 2,6-tolylene diisocyanate, 1,3-xylylene diisocyanate, 1,4-xylylene diisocyanate, diphenylmethane-4,4'-diisocyanate, and the like. Examples of the above-mentioned (meth)acrylate having a hydroxyl group include 2-hydroxy(meth)acrylate, hydroxypropyl (meth)acrylate, 4-hydroxybutyl (meth)acrylate, and the like. Examples of the (meth)acrylate having an isocyanate group include methacryloyloxyethylisocyanate.

Examples of the commercially available product of the above-mentioned urethane (meth)acrylate (B1) include product names: SHIKOH series UV-2000B, UV-6640B, UV-3000B, UV-3200B, UV-3300B, and UV-3700B, which are manufactured by Mitsubishi Chemical Corporation, product names: EBECRYL230, EBECRYL4491, EBECRYL8411, and KRM8961, which are manufactured by Daicel-Allnex Ltd.,, product names: SUA-008 and SUA-074, which are manufactured by Asia Industry Co., Ltd., and the like.

The amount of urethane (meth)acrylate (B1) contained in the photocurable resin composition of the present invention is 0 to 70% by mass, preferably 5 to 50% by mass, more preferably 10 to 30% by mass, with respect to the total of the monomer components (100% by mass) of the photocurable resin composition, from the aspects of the viscosity of the resin composition before curing and the flexibility of the film obtained after curing.

### [Monomer (C) (component (C))]

The photocurable resin composition of the present invention may further contain monomer (C) other than di(meth)acrylate (A) and urethane (meth)acrylate (B1). As the monomer (C), for example, those having a radical polymerizable functional group such as acryloyl group, methacryloyl group, or the like as the reactive functional group are preferred. Examples of the monomer (C) include (meth)acrylate esters of monoalcohol (hydroxypropyl methacrylate and the like). As monomer (C), a monofunctional acrylate having an alkyl group, an aromatic ring, an alicyclic ring, and the like, which are generally called reactive diluents, may also be used. The content of monomer (C) in the photocurable resin composition of the present invention is preferably 0 to 30% by mass, more preferably 5 to 25% by mass, further preferably 10 to 20% by mass, with respect to the total of the monomer components (100% by mass) of the photocurable resin composition, from the aspects of the viscosity of the resin composition before curing and the bending resistance of the film obtained after curing.

### [Photopolymerization initiator (D) (component (D))]

The photocurable resin composition of the present invention may further contain photopolymerization initiator (D). The photopolymerization initiator (D) is activated by the action of light and starts curing of the photocurable compounds such as the above-mentioned component (A), component (B1), component (C), and the like. Examples of the photopolymerization initiator (D) include radical polymerization initiators that generate radicals by the action of light, as well as those that generate acids (or cations) by the action of light. Only one kind of photopolymerization initiator (D) may be used or two or more kinds thereof may be used in combination. The photopolymerization initiator (D) is selected according to the type of photocurable compound, for example, whether it is radically polymerizable or cationically polymerizable, and the like. Examples of the radical photopolymerization initiator include alkylphenone-based photopolymerization initiator, acylphosphine oxide-based photopolymerization initiator, and the like.

Examples of the alkylphenone-based photopolymerization initiator include 2,2-dimethoxy-1,2-diphenylethan-1-one (IGM RESINS B.V., Omnirad 651), 1-hydroxy-cyclohexyl-phenyl-ketone (IGM RESINS B.V., Omnirad 184), 2-hydroxy-2-methyl-1-phenylpropan-1-one (IGM RESINS B.V., Omnirad 1173), 1-[4-(2-hydroxyethoxy)-phenyl]-2-hydroxy-2-methyl-1-propan-1-one (IGM RESINS B.V., Omnirad 2959), 2-hydroxy-1-{4-[4-(2-hydroxy-2-methyl-propionyl)-benzyl]phenyl}-2-methyl-propan-1-one (IGM RESINS B.V., Omnirad 127), 2-methyl-1-(4-methylthiophenyl)-2-morpholinopropan-1-one (IGM RESINS B.V., Omnirad 907), 2-benzyl-2-dimethylamino-1-(4-morpholinophenyl)-butanone-1 (IGM RESINS B.V., Omnirad 369E), 2-(dimethylamino)-2-[(4-methylphenyl)methyl]-1-[4-(4-morpholinyl)phenyl]-1-butanone (IGM RESINS B.V., Omnirad 379EG), and the like.

Examples of the acylphosphine oxide-based photopolymerization initiator include 2,4,6-trimethylbenzoyldiphenyl-phosphine oxide (IGM RESINS B.V., Omnirad TPO), bis(2,4,6-trimethylbenzoyl)-phenylphosphine oxide (IGM RESINS B.V., Omnirad 819), and the like.

The content of the photopolymerization initiator (D) in the photocurable resin composition of the present invention is not particularly limited as long as it starts curing of photocurable compounds such as the above-mentioned component (A), component (B1), component (C), and the like. It is preferably 0.1 to 20 parts by mass with respect to the total of the di(meth)acrylate (A), urethane (meth)acrylate (B1), and monomer (C) (100 parts by mass) in the resin composition. From the aspects of the curability of the photocurable resin composition and the property of the obtained cured product, it is more preferably 0.3 to 10 parts by mass, further preferably 0.5 to 5 parts by mass.

### [Others]

The photocurable resin composition of the present invention may also contain other known photocurable resins and the like. In addition, the photocurable resin composition of the present invention can contain known additives.

The photocurable resin composition of the present invention is cured by light irradiation (for example, ultraviolet irradiation) to become a cured product. The photocurable resin composition of the present invention is in a liquid form and can be easily applied and shaped using various coaters, inkjet printers, 3D printers, and the like. The viscosity of the photocurable resin composition at 25°C in this case is, for example, not less than 5 mPa·s and not more than 5Pa·s (specifically, it can be measured, for example, by the method described in Example below). It is preferably liquid and homogeneous at room temperature (e.g., not less than 20°C and not more than 35°C). Assuming use in winter, it is preferable that the composition should maintain flowability and transparency even at low temperatures because the composition cannot be used if it becomes cloudy or solidifies at around 0°C.

When whitening or the like occurs in a cured film made of a cured product of a photocurable resin composition, the appearance is impaired during coating, and therefore, a transparent appearance of the film after curing is preferred. Transparency is determined by measuring the transparency of the cured film using a haze meter (specifically, it can be evaluated, for example, by the method described in the Examples below). The cured film exhibits high bending resistance, and even when bent, does not cause cracks or discoloration and the like of the bent portion. The bending resistance is determined by wrapping the cured film around SUS rods with diameters of 10 mm and 15 mm, and visually observing changes in appearance (specifically, it can be evaluated, for example, by the method described in the Examples below). The obtained cured film has resistance in normal use, with no change in appearance when exposed to humidity or water. Water resistance is determined from changes in the film before and after exposure to water for 24 hr (specifically, it can be evaluated, for example, by the method described in the Examples below).

### [Photocurable resin composition for adhesive]

The photocurable resin composition for adhesive of the present invention contains component (A): di(meth)acrylate (A) represented by the formula (1), component (B): polymerizable oligomer (B) having two or more (meth)acryloyl groups in one molecule, and component (C1): monofunctional (meth)acrylate monomer (C1), and is characterized in that the content of component (A) is 1 to 50% by mass, the content of component (B) is 15 to 98% by mass, and the content of component (C1) is 1 to 35% by mass, with respect to the total of the component (A), component (B) and component (C1) (100% by mass).

### [di(meth)acrylate (A) (component (A))]

The di(meth)acrylate (A) is a polytrimethylene glycol di(meth)acrylate represented by the following formula (1).

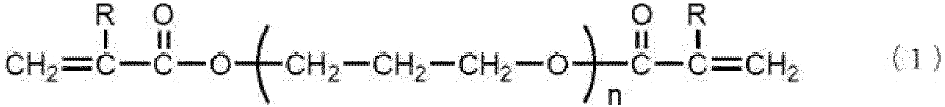

In the formula, R is a hydrogen atom or a methyl group.

In the formula, n is an average number of moles of added oxytrimethylene group and is a real number of 5 to 90. From the aspects of the coatability (particularly coatability onto concave-convex surfaces) of the resin composition before curing, and the transparency and water-resistant adhesiveness of the cured product (adhesive layer), n is preferably a real number of 7 to 50, more preferably a real number of 8 to 40.

The di(meth)acrylate (A) and a production method thereof are as described in the above-mentioned [Photocurable resin composition].

Only one kind of di(meth)acrylate (A) represented by the formula (1) may be used alone or two or more kinds thereof may be used in combination.

The amount of the di(meth)acrylate (A) contained in the photocurable resin composition for adhesive of the present invention is 1 to 50% by mass, preferably 10 to 45% by mass, more preferably 15 to 40% by mass, with respect to a total amount of component (A), component (B) and component (C1) (100% by mass), from the aspects of the flexibility of the cured product (adhesive layer) during bending and adhesiveness thereof to substrates with concaves and convexes.

### [Polymerizable oligomer (B) (component (B))]

The polymerizable oligomer (B) in the present invention has two or more (meth)acryloyl groups in one molecule and preferably has a weight average molecular weight of 500 to 100000. The number of (meth)acryloyl groups is preferably 2 to 3, more preferably 2, from the aspects of the adhesiveness of the cured product (adhesive layer) to a substrate with concaves and convexes and the flexibility during bending. The weight average molecular weight can be determined based on polystyrene by using, for example, gel permeation chromatography (GPC). Only one kind of the polymerizable oligomer (B) may be used alone or two or more kinds thereof may be used in combination.

The structure of the polymerizable oligomer (B) is not particularly limited. Examples thereof include urethane (meth)acrylate oligomer, epoxy (meth)acrylate oligomer, polyester (meth)acrylate oligomer, and the like, and urethane (meth)acrylate oligomer is preferred from the aspects of the adhesiveness of the cured product (adhesive layer) to a substrate with concaves and convexes and the flexibility during bending.

The urethane (meth)acrylate oligomer means a compound having a urethane bond and a (meth)acryloyl group in one molecule. The method for producing the urethane (meth)acrylate oligomer is not particularly limited. For example, it can be obtained by reacting a polyhydric alcohol, a polyisocyanate such as isocyanate or isocyanurate, and a (meth)acrylate having a hydroxyl group or a (meth)acrylate having an isocyanate group.

Example of the above-mentioned polyhydric alcohol include polyhydric alcohols having a polyether skeleton, polyester skeleton, or polycarbonate skeleton in the molecule, in addition to aromatic, aliphatic, and alicyclic polyols. As the aliphatic and alicyclic polyols, ethylene glycol, propylene glycol, trimethylene glycol, 1,4-cyclohexanediol, 1,4-cyclohexanedimethanol, and the like can be mentioned. As the polyhydric alcohol having a polyether skeleton, polyalkylene glycols such as polyethylene glycol, polypropylene glycol, polytetramethylene glycol, and the like can be mentioned. Polyhydric alcohol having a polyester skeleton is obtained by an esterification reaction between the aforementioned polyhydric alcohol and polybasic carboxylic acid or an anhydride thereof and, as the polybasic carboxylic acid, succinic acid, succinic anhydride, adipic acid, maleic acid, maleic anhydride, and the like can be mentioned. As the polyhydric alcohol having a polycarbonate skeleton, those obtained by the reaction of the aforementioned polyhydric alcohol with a carbonate compound such as dialkyl carbonate, alkylene carbonate, diphenyl carbonate, or the like can be mentioned.

As the polyhydric alcohol, polyhydric alcohol having a polyether skeleton or a polyester skeleton is preferred, and polyhydric alcohol having a polyether skeleton is particularly preferred. As the polyhydric alcohol having a polyether skeleton, polyhydric alcohol having a polyalkylene glycol skeleton having 2 to 4 carbon atoms is preferred, and polyhydric alcohol having a polypropylene glycol skeleton is more preferred.

Examples of the above-mentioned polyisocyanate include aromatic diisocyanates such as isophorone diisocyanate, 2,4-tolylene diisocyanate, 2,6-tolylene diisocyanate, 1,3-xylylene diisocyanate, 1,4-xylylene diisocyanate, diphenylmethane-4,4'-diisocyanate, and the like, aliphatic diisocyanates such as hexamethylene diisocyanate, trimethylhexamethylene diisocyanate, and the like, alicyclic diisocyanates such as 1,4-cyclohexane diisocyanate, norbornene diisocyanate, and the like. As the polyisocyanate, diisocyanate is preferred, and diisocyanate having a cyclic structure is more preferred.

Examples of the above-mentioned (meth)acrylate having a hydroxyl group include 2-hydroxyethyl (meth)acrylate, 2-hydroxypropyl (meth)acrylate, 2-hydroxybutyl (meth)acrylate, 4-hydroxybutyl (meth)acrylate, 1,4-cyclohexanedimethanol monoacrylate, and the like. Examples of the (meth)acrylate having an isocyanate group include methacryloyloxyethylisocyanate. As the (meth)acrylate having a hydroxyl group or an isocyanate group, the (meth)acrylate having a hydroxyl group is preferred, and 2-hydroxyethyl (meth)acrylate, 2-hydroxypropyl (meth)acrylate, 2-hydroxybutyl (meth)acrylate, and 4-hydroxybutyl (meth)acrylate are more preferred.

As the urethane (meth)acrylate oligomer, polyether, polyester, polycarbonate oligomers can be mentioned. From the aspects of the flexibility of the cured product (adhesive layer) during bending and adhesiveness thereof to substrates with concaves and convexes, polyether urethane (meth)acrylate oligomers and polyester urethane (meth)acrylate oligomers are preferred, and polyether urethane (meth)acrylate oligomers are more preferred. The weight average molecular weight is preferably 1000 to 70000, more preferably 5000 to 40000, from the aspects of the coatability (particularly coatability onto concave-convex surfaces) of the resin composition before curing, and the flexibility of the cured product (adhesive layer) during bending. The glass transition temperature of the cured product obtained by curing urethane (meth)acrylate oligomer alone is preferably not more than 20°C, more preferably not more than 10°C, further preferably not more than 0°C, from the aspect of obtaining sufficient flexibility at room temperature of a cured product (adhesive layer) of the photocurable resin composition for adhesive. The glass transition temperature can be measured using, for example, a differential scanning calorimeter.

Examples of the commercially available product of the urethane (meth)acrylate oligomer include product names: UV-3300B, UV-3700B, and UV-6640B, which are manufactured by Mitsubishi Chemical Corporation, and product names: UN-6200, UN-6207, and UN-6060S, which are manufactured by Negami Chemical Industrial Co., Ltd., as the urethane (meth)acrylate having a polyether skeleton, and product names: UV-3000B and UV-3200B, which are manufactured by Mitsubishi Chemical Corporation, and product names: UN-7700 and UN-7600, which are manufactured by Negami Chemical Industrial Co., Ltd., as the urethane (meth)acrylate having a polyester skeleton, and the like.

The epoxy (meth)acrylate oligomer means a compound having an epoxy group and a (meth)acryloyl group in one molecule. While the method for producing the epoxy (meth)acrylate oligomer is not particularly limited, it can be obtained by reacting the epoxy group of a compound having an epoxy group with a (meth)acrylate having a carboxyl group or a hydroxyl group. For example, an epoxy (meth)acrylate oligomer, which is a bisphenol type A epoxy compound, can be synthesized by reacting bisphenol type A diglycidyl ether with a compound having one or more carboxyl groups in the molecule, such as (meth)acrylic acid. The epoxy (meth)acrylate oligomer may have a small amount of epoxy group remaining after curing of the photocurable resin composition for adhesive. However, it is preferable that the epoxy group should be completely consumed during curing of the photocurable resin composition for adhesive. Examples of the commercially available product of the epoxy (meth)acrylate oligomer include product names: CN104 and CN111, which are manufactured by SARTOMER, product names: EBECRYL600, EBECRYL3708, and EBECRYL860, which are manufactured by Daicel-Allnex Ltd., and the like.

The polyester (meth)acrylate oligomer means a compound having an ester bond and a (meth)acryloyl group in one molecule. The method for producing the polyester (meth)acrylate oligomer is not particularly limited. For example, it is obtained by an esterification reaction between the hydroxyl group of a polyester synthesized by polycondensation of a polybasic acid and a polyhydric alcohol with (meth)acrylate having a carboxyl group. Examples of the polybasic acid include aliphatic polybasic acids such as oxalic acid, malonic acid, succinic acid, maleic acid, itaconic acid, succinic anhydride, and the like, alicyclic polybasic acids such as dimer acid, cyclohexanedicarboxylic acid, and the like, aromatic polybasic acids such as isophthalic acid, terephthalic acid, and the like. As the polyhydric alcohol, polyol is preferred, and examples thereof include ethylene glycol, diethylene glycol, propylene glycol, 3-methyl-1,5-pentanediol, cyclohexanediol, 1,3-bis(2-hydroxyethoxy)benzene, 4,4'-methylenediphenol, polyoxyethylene glycol, polyoxypropylene glycol, and the like. Examples of the commercially available product of the polyester (meth)acrylate oligomer include product names:CN296, CN2203, CN2259, and CN2270, which are manufactured by SARTOMER, and the like.

The amount of the polymerizable oligomer (B) contained in the photocurable resin composition for adhesive of the present invention is 15 to 98% by mass, preferably 25 to 70% by mass, more preferably 40 to 60% by mass, with respect to a total amount of component (A), component (B) and component (C1) (100% by mass), from the aspects of coatability of the resin composition before curing onto concave-convex surfaces and flexibility of the cured product (adhesive layer) during bending.

### [Monofunctional (meth)acrylate monomer (C1) (component (C1))]

The monofunctional (meth)acrylate monomer (C1) is not particularly limited as long as it is a (meth)acrylate compound having one (meth)acryloyl group in one molecule. One kind of monofunctional (meth)acrylate monomer (C1) may be used alone, or two or more kinds thereof may be used in combination.

Examples of the monofunctional (meth)acrylate monomer (C1) include methyl (meth)acrylate, ethyl (meth)acrylate, propyl (meth)acrylate, butyl (meth)acrylate, hexyl (meth)acrylate, octyl (meth)acrylate, 2-ethylhexyl (meth)acrylate, nonyl (meth)acrylate, dodecyl (meth)acrylate, stearyl (meth)acrylate, 2-methoxyethyl (meth)acrylate, 3-methoxybutyl (meth)acrylate, cyclohexyl (meth)acrylate, isobornyl (meth)acrylate, phenyl (meth)acrylate, benzyl (meth)acrylate, 2-hydroxyethyl (meth)acrylate, 2-hydroxypropyl (meth)acrylate, 4-hydroxybutyl (meth)acrylate, glycerol mono(meth)acrylate, hydroxyphenyl (meth)acrylate, glycidyl (meth)acrylate, and the like. From the aspects of the flexibility of the cured product (adhesive layer) during bending, adhesiveness thereof to substrates with concaves and convexes, and curability of the photocurable resin composition for adhesive, propyl (meth)acrylate, butyl (meth)acrylate, 2-hydroxyethyl (meth)acrylate, 2-hydroxypropyl (meth)acrylate, and 4-hydroxybutyl (meth)acrylate are preferred, and propyl acrylate, butyl acrylate, 2-hydroxyethyl acrylate, 2-hydroxypropyl acrylate, and 4-hydroxybutyl acrylate are more preferred.

The amount of the monofunctional (meth)acrylate monomer (C1) contained in the photocurable resin composition for adhesive of the present invention is 1 to 35% by mass, preferably 10 to 30% by mass, more preferably 15 to 25% by mass, with respect to a total amount of component (A), component (B), and component (C1) (100% by mass), from the aspects of coatability (particularly coatability onto concave-convex surfaces) and curability of the photocurable resin composition for adhesive.

### [Photopolymerization initiator (D) (component (D))]

The photocurable resin composition for adhesive of the present invention may further contain photopolymerization initiator (D). The photopolymerization initiator (D) is as described in the above-mentioned [Photocurable resin composition].

The content of the photopolymerization initiator (D) in the photocurable resin composition for adhesive of the present invention is not particularly limited as long as it starts curing of photocurable compounds such as the component (A), component (B), component (C1), and the like. It is preferably 0.1 to 20 parts by mass with respect to the total of the component (A), component (B), and component (C1) (100 parts by mass) in the resin composition. From the aspect of the curability of the photocurable resin composition for adhesive, it is more preferably 0.3 to 10 parts by mass, further preferably 0.5 to 5 parts by mass.

### [Others]

The photocurable resin composition for adhesive of the present invention may further contain known photocurable resins and solvents as long as the effects of the present invention are not impaired. In addition, it may contain known additives such as antioxidant, polymerization inhibitor, light stabilizer, plasticizer, thickener, tackifier, antifoaming agent, ultraviolet absorber, and the like. The contents of these are preferably 0 to 20 parts by mass, more preferably 0 to 15 parts by mass, further preferably 0 to 10 parts by mass, with respect to the total of the component (A), component (B), and component (C1) (100 parts by mass) in the photocurable resin composition for adhesive.

The photocurable resin composition for adhesive of the present invention can be prepared by mixing respective components, and the method and order of mixing are not particularly limited. For mixing, various containers and stirring blades, stirrers, and the like can be used.

For example, when a transparent substrate is adhered, the photocurable resin composition for adhesive of the present invention is applied to one of the substrates by using a coating device such as a slit coater, roll coater, spin coater, or screen printing, the other substrate is adhered to this coated surface, and cured by irradiating light from the transparent substrate side, whereby a cured product (adhesive layer) can be produced. As the curing light, ultraviolet light (wavelength around 200 to 400 nm) and known light sources that emit such light can be used.

### [Example]

The present invention is explained in detail in the following by referring to Examples and Comparative Examples, which are not to be construed as limiting the present invention.

### [Synthetic Example]

### Synthesis of di(meth)acrylate A-1 (component (A-1))

Into a 2 L 5-necked flask with air inlet tube, impeller, thermometer, and oil/water separator were added toluene (manufactured by Kishida Chemical Co., Ltd., reagent special grade) (420 g), polytrimethylene glycol (manufactured by ALLESSA, Velvetol H-500 [molecular weight 500]) (500 g), methacrylic acid (manufactured by Tokyo Chemical Industry Co., Ltd.) (190 g), paratoluenesulfonic acid (manufactured by Kishida Chemical Co., Ltd., reagent special grade) (82 g), and 4-methoxyphenol (manufactured by Kishida Chemical Co., Ltd., reagent special grade) (1 g), and the mixture was heated to 100°C and dehydrated and esterified at 300 torr for 10 hr. The obtained reaction mixture was cooled to room temperature, washed with 20% sodium hydroxide aqueous solution (300 g) and, after discharging the lower water layer, washed with 20% brine (240 g). Toluene was evaporated under reduced pressure at 60°C, 20 torr to obtain di(meth)acrylate A-1 at yield 80%. acid value 0.2 mgKOH/g, hydroxyl value 2.0 mgKOH/g, saponification value 173 mgKOH/g

### Synthesis of di(meth)acrylate A-2 ((A-2) component)

By synthesizing in the same manner as in A-1 except that polytrimethylene glycol (manufactured by ALLESSA, Velvetol H-500) was changed to polytrimethylene glycol (manufactured by ALLESSA, Velvetol H-1000 [molecular weight 1000]), methacrylic acid was changed to 95 g, and paratoluenesulfonic acid was changed to 41 g, di(meth)acrylate A-2 was obtained at yield 75%. acid value 0.1 mgKOH/g, hydroxyl value 3.1 mgKOH/g, saponification value 95 mgKOH/g

### Synthesis of di(meth)acrylate A-3 (component (A-3))

By synthesizing in the same manner as in A-1 except that polytrimethylene glycol (manufactured by ALLESSA, Velvetol H-500) was changed to polytrimethylene glycol (manufactured by ALLESSA, Velvetol H-2000 [molecular weight 2000]), methacrylic acid was changed to 50 g, paratoluenesulfonic acid was changed to 25 g, di(meth)acrylate A-3 was obtained at yield 63%. acid value 0.3 mgKOH/g, hydroxyl value 4.1 mgKOH/g, saponification value 50 mgKOH/g

### Synthesis of di(meth)acrylate A-4 (component (A-4))

By synthesizing in the same manner as in A-1 except that polytrimethylene glycol (manufactured by ALLESSA, Velvetol H-500) was changed to polytrimethylene glycol (manufactured by ALLESSA, Velvetol H-2700 [molecular weight 2700]), methacrylic acid was changed to 35 g, paratoluenesulfonic acid was changed to 18 g, di(meth)acrylate A-4 was obtained at yield 610. acid value 0.5 mgKOH/g, hydroxyl value 4.9 mgKOH/g, saponification value 38 mgKOH/g

### di(meth)acrylate A-5 (component (A-5))

Blemmer PDE-600 (manufactured by NOF CORPORATION) was used.

### Synthesis of di(meth)acrylate A-6 (component (A-6))

By synthesizing in the same manner as in A-1 except that polytrimethylene glycol (manufactured by ALLESSA, Velvetol H-500) was changed to polypropylene glycol (manufactured by FUJIFILM Wako Pure Chemical Corporation, polypropylene glycol, diol type, 1000 [molecular weight 1000]), methacrylic acid was changed to 95 g, and paratoluenesulfonic acid was changed to 41 g, di(meth)acrylate A-6 was obtained at yield 89%. acid value 0.1 mgKOH/g, hydroxyl value 3.5 mgKOH/g, saponification value 90 mgKOH/g

### Synthesis of di(meth)acrylate A-7 (component (A-7))

By synthesizing in the same manner as in A-1 except that polytetramethylene glycol (manufactured by ALLESSA, Velvetol H-500) was changed to polytetramethylene glycol (manufactured by FUJIFILM Wako Pure Chemical Corporation, polytetramethylene oxide 1,000 [molecular weight 1000]), methacrylic acid was changed to 95 g, and paratoluenesulfonic acid was changed to 41 g, di(meth)acrylate A-7 was obtained at yield 910. acid value 0.1 mgKOH/g, hydroxyl value 1.5 mgKOH/g, saponification value 94 mgKOH/g

### Synthesis of di(meth)acrylate A-8 (component (A-8))

By synthesizing in the same manner as in A-1 except that polytrimethylene glycol (manufactured by ALLESSA, Velvetol H-500) was changed to polytrimethylene glycol (manufactured by ALLESSA, Velvetol H-1000), acrylic acid was changed to 80 g, and paratoluenesulfonic acid was changed to 41 g, di(meth)acrylate A-8 was obtained at yield 55%. acid value 0.3 mgKOH/g, hydroxyl value 4.2 mgKOH/g, saponification value 98 mgKOH/g

### di(meth)acrylate A-9 (component (A-9))

Blemmer PDP-400N (manufactured by NOF CORPORATION) was used.

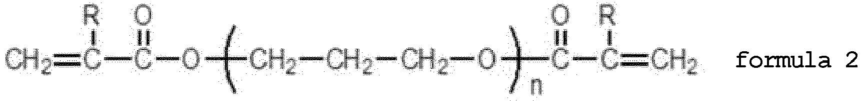

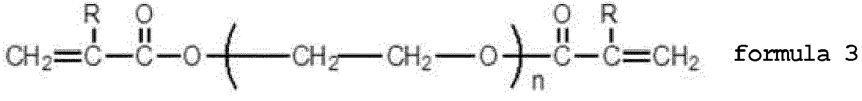

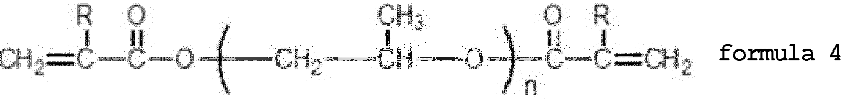

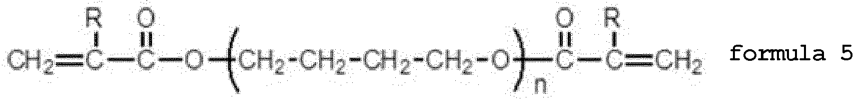

**[Table 1]**

| di(meth)acrylate | abbreviation | | formula | average number of moles added (n) | R |
|---|---|---|---|---|---|
| A-1 | | PDV-500 | 2 | 9 | CH₃ |
| A-2 | | PDV-1000 | 2 | 17 | CH₃ |
| A-3 | | PDV-2000 | 2 | 34 | CH₃ |
| A-4 | | PDV-2700 | 2 | 47 | CH₃ |
| A-5 | | PDE-600 | 3 | 14 | CH₃ |
| A-6 | | PDP-1000 | 4 | 17 | CH₃ |
| A-7 | | PDT-1000 | 5 | 14 | CH₃ |
| A-8 | | ADV-1000 | 2 | 17 | H |
| A-9 | | PDP-400 | 4 | 7 | CH₃ |

### urethane (meth)acrylate B1-1 (component (B1-1))

UV-3300B: manufactured by Mitsubishi Chemical Corporation, SHIKOH UV-3300B (weight average molecular weight 13000, number of acryloyl group 2)

### monomer C-1 (component (C-1))

HPMA: hydroxypropyl methacrylate

photopolymerization initiator D-1 (component (D-1))

### Omnirad 651 (2,2-dimethoxy-2-phenylacetophenone)

### polymerizable oligomer B-1 (component (B-1))

UV-3700B: urethane acrylate oligomer (manufactured by Mitsubishi Chemical Corporation, weight average molecular weight 38000, number of acryloyl group 2)

### polymerizable oligomer B-2 (component (B-2))

EBECRYL3708: epoxy acrylate oligomer (manufactured by Daicel-Allnex Ltd., weight average molecular weight 1500, number of acryloyl group 2)

### monofunctional (meth)acrylate monomer C1-1 (component (C1-1))

4HBA: 4-hydroxybutyl acrylate

### [Preparation of photocurable resin composition]

Di(meth)acrylate (component (A)), urethane (meth)acrylate (component (B1)), and monomer (component (C)) were added to a total of 20 g in the formulations shown in Table 2 (Example) and Table 3 (Comparative Example), 0.6 g of Omnirad 651 was added as a photopolymerization initiator, and the mixture was stirred at 60°C. In this way, photocurable resin compositions of Examples and Comparative Examples were prepared.

**[Table 2]**

| component | Example | | | | | |
|---|---|---|---|---|---|---|
| | 1 | 2 | 3 | 4 | 5 | 6 |
| A-1 (PDV-500) | 70 | - | - | - | - | - |
| A-2 (PDV-1000) | - | 70 | - | - | 40 | 100 |
| A-3 (PDV-2000) | - | - | 70 | - | - | - |
| A-4 (PDV-2700) | - | - | - | 70 | - | - |
| A-5 (PDE-600) | - | - | - | - | - | - |
| A-6 (PDP-1000) | - | - | - | - | - | - |
| A-7 (PDT-1000) | - | - | - | - | - | - |
| B1-1 (UV-3300B) | 15 | 15 | 15 | 15 | 45 | - |
| C-1 (HPMA) | 15 | 15 | 15 | 15 | 15 | - |
| D-1 (Omnirad 651) | 3 | 3 | 3 | 3 | 3 | 3 |

**[Table 3]**

| component | Comparative Example | | | |
|---|---|---|---|---|
| | 1 | 2 | 3 | 4 |
| A-1 (PDV-500) | - | - | - | - |
| A-2 (PDV-1000) | - | - | - | - |
| A-3 (PDV-2000) | - | - | - | - |
| A-4 (PDV-2700) | - | - | - | - |
| A-5 (PDE-600) | 70 | - | - | - |
| A-6 (PDP-1000) | - | 70 | - | - |
| A-7 (PDT-1000) | - | - | 70 | - |
| B1-1 (UV-3300B) | 15 | 15 | 15 | 85 |
| C-1 (HPMA) | 15 | 15 | 15 | 15 |
| D-1 (Omnirad 651) | 3 | 3 | 3 | 3 |

In Tables 2 and 3, the amounts of component (A), component (B1), and component (C) added are expressed as a percentage by mass of each component relative to 100 mass% of the total amount of these components. The amount of a photopolymerization initiator added is expressed as parts by mass relative to the total amount 100 parts by mass of component (A), component (B1), and component (C).

### [Measurement of viscosity of photocurable resin composition]

The viscosity at 25°C of the photocurable resin composition prepared in the above was measured by E type viscosimeter (manufactured by Toki Sangyo Co., Ltd., TV-25).
⊙: less than 500 mPa·s
○: not less than 500 mPa·s and less than 3000 mPa·s
Δ: not less than 3000 mPa·s

### [Low temperature storage]

The photocurable resin composition before curing was placed in a thermostatic bath at 5°C, left standing for 24 hr, and the condition of the photocurable resin composition was visually evaluated according to the following criteria.
O: Transparent with no precipitate or cloudiness
Δ: Precipitate or cloudiness is observed.

### [Curing of photocurable resin composition]

A photocurable resin composition prepared in the above was injected into glass plates sandwiching a silicone spacer with film thickness 1 mm. The photocurable resin composition was cured by irradiating UV light with a UV lamp at a cumulative light intensity of 1000 mJ/cm² to obtain a test piece cured film (100×500×10 mm) .

### [Transparency of film]

The transparency of the obtained test piece cured film was measured with a haze meter, and evaluated according to the following criteria.
⊙: haze less than 0.1
○: haze not less than 0.1 and less than 1.0
Δ: haze not less than 1.0

### [Evaluation of bending resistance of photocurable resin composition after curing]

The obtained test piece cured film was wrapped around SUS rods with diameters of 10 mm and 15 mm, and changes in the appearance were visually evaluated according to the following criteria.
⊙: Cured film wrapped around SUS rods with diameter 10 mm and diameter 15 mm shows no crack.
○: Cured film wrapped around SUS rod with diameter 10 mm shows crack and cured film wrapped around SUS rod with diameter 15 mm shows no crack.
Δ: Cured film wrapped around SUS rods with diameter 10 mm and diameter 15 mm shows crack.

### [Water resistance of cured film]

In a 100 mL beaker was placed 90 g of ion exchange water, and the test piece cured film was immersed therein, left standing at 25°C for 24 hr, and the condition of the film was visually evaluated according to the following criteria.
⊙: No change in appearance
○: Swelling and cloudiness are seen in some parts
Δ: Swelling and cloudiness are seen in the entirety

The results obtained in the above-mentioned tests are shown in Tables 4 (Example) and 5 (Comparative Example).

**[Table 4]**

| | Comparative Example | | | |
|---|---|---|---|---|
| | 1 | 2 | 3 | 4 |
| viscosity (mPa·s, 25°C) | ⊙ (95) | ⊙ (98) | ○ (690) | Δ (4040) |
| storage at low temperature | ○ | ○ | Δ | Δ |
| water resistance | Δ | ⊙ | ⊙ | ⊙ |
| bending resistance | Δ | Δ | ⊙ | ⊙ |
| transparency of film (haze) | ○ (0.15) | Δ (1.1) | Δ (4.9) | ⊙ (0.08) |

**[Table 5]**

| | Comparative Example | | | |
|---|---|---|---|---|
| | 1 | 2 | 3 | 4 |
| viscosity (mPa·s, 25°C) | ⊙ (95) | ⊙ (98) | ○ (690) | Δ (4040) |
| storage at low temperature | ○ | ○ | Δ | Δ |
| water resistance | Δ | ⊙ | ⊙ | ⊙ |
| bending resistance | Δ | Δ | ⊙ | ⊙ |
| transparency of film (haze) | ○ (0.15) | Δ (1.1) | Δ (4.9) | ⊙ (0.08) |

From the above-mentioned results, it can be seen that the photocurable resin compositions (Examples 1 to 6) using di(meth)acrylate represented by the formula (1) (A-1 to A-4) in the present invention have low viscosity before curing, are superior in storage at low temperatures, and afford after curing films superior in water resistance, bending resistance, and transparency.

### [Preparation of photocurable resin composition for adhesive]

Di(meth)acrylate (component (A)), polymerizable oligomer (component (B)), and monofunctional (meth)acrylate monomer (component (C1)) were added to a total of 20 g in the formulations shown in Table 6, 0.6 g of Omnirad 651 was added as a photopolymerization initiator (component (D)), and the mixture was stirred at 60°C. In this way, photocurable resin compositions for adhesive of Examples and Comparative Examples were prepared.

**[Table 6]**

| component | Example | | | | | | | Comparative Example | | | |
|---|---|---|---|---|---|---|---|---|---|---|---|
| | 7 | 8 | 9 | 10 | 11 | 12 | 13 | 5 | 6 | 7 | 8 |
| A-1 (PDV-500) | 30 | - | - | - | - | - | - | - | - | - | - |
| A-2 (PDV-1000) | - | 30 | - | - | 30 | 5 | 50 | - | - | - | - |
| A-4 (PDV-2700) | - | - | 30 | - | - | - | - | - | - | - | - |
| A-8 (ADV-1000) | - | - | - | 30 | - | - | - | - | - | - | - |
| A-5 (PDE-600) | - | - | - | - | - | - | - | 30 | - | - | - |
| A-9 (PDP-400) | - | - | - | - | - | - | - | - | 30 | - | - |
| A-7 (PDT-1000) | - | - | - | - | - | - | - | - | - | 30 | - |
| B-1 (UV-3700B) | 50 | 50 | 50 | 50 | - | 75 | 30 | 50 | 50 | 50 | 80 |
| B-2 (EBECRYL3708) | - | - | - | - | 50 | - | - | - | - | - | - |
| C1-1 (4HBA) | 20 | 20 | 20 | 20 | 20 | 20 | 20 | 20 | 20 | 20 | 20 |
| D-1 (Omnirad 651) | 3 | 3 | 3 | 3 | 3 | 3 | 3 | 3 | 3 | 3 | 3 |

In Table 6, the amounts of component (A), component (B), and component (C1) added are expressed as a percentage by mass of each component relative to 100 mass% of the total amount of these components. The amount of component (D) added is expressed as parts by mass relative to the total 100 parts by mass of component (A), component (B), and component (C1).

### [Evaluation of transparency]

A photocurable resin composition for adhesive prepared in the above was injected into glass plates sandwiching a silicone spacer with film thickness 300 µm. The photocurable resin composition for adhesive was cured by irradiating UV light with a UV lamp at a cumulative light intensity of 1000 mJ/cm² to obtain a test piece of an adhesive layer (100×500×0.3 mm). The transparency of the obtained adhesive layer test piece was measured with a haze meter and evaluated according to the following criteria.
⊙: haze less than 0.1
○: haze not less than 0.1 and less than 1.0
Δ: haze not less than 1.0

### [Evaluation of coatability onto concave-convex surfaces]

The photocurable resin composition for adhesive prepared in the above was dropped onto an embossed PP film (clear file manufactured by King Jim Co., Ltd., 25 mm × 100 mm × thickness 0.05 mm), and a non-embossed PET film (A4160 manufactured by Toyobo Co., Ltd.) was quickly laminated thereon. The photocurable resin composition for adhesive was cured by irradiating UV light with a UV lamp at a cumulative light intensity of 1000 mJ/cm² to obtain a test piece with the film laminated thereon. The presence or absence of air bubbles in the bonded portion (adhesive layer) of the obtained film test piece was visually observed and evaluated according to the following criteria.
○: No bubble is observed.
Δ: Bubbles are seen in some parts.
×: Many bubbles are observed.

### [Evaluation of adhesiveness]

A film test piece obtained by a curing method similar to the above was bent 180 degrees along an SUS rod with diameter 5 mm to perform the test. This test was repeated five times, and changes in appearance after the test were visually evaluated according to the following criteria.
⊙: No delamination of cured film (adhesive layer) even after bending 5 times.
○: No delamination of cured film (adhesive layer) after bending once, but delamination was found after bending 5 times.
Δ: Delamination of cured film (adhesive layer) after bending once.

### [Evaluation of flexibility]

A test similar to the above-mentioned evaluation of adhesiveness was performed, and changes in appearance after the test were visually evaluated according to the following criteria.
⊙: No cracking of cured film (adhesive layer) even after bending 5 times.
O: No cracking of cured film (adhesive layer) after bending once, but cracking was found after bending 5 times.
Δ: Cracking of cured film (adhesive layer) after bending once.

### [Evaluation of water-resistant adhesiveness]

The photocurable resin composition for adhesive prepared in the above was dropped onto a PET film (A4160 manufactured by Toyobo Co., Ltd.), a similar PET film was quickly laminated thereon. The photocurable resin composition for adhesive was cured by irradiating UV light with a UV lamp at a cumulative light intensity of 1000 mJ/cm² to obtain a test piece with the film laminated thereon. The obtained PET film test piece was stored at 80°C, humidity 90% for 24 hr. The test piece after storage was bent 180 degrees along an SUS rod with diameter 5 mm to perform the test. The water-resistant adhesiveness was evaluated according to the following criteria.
O: No delamination of cured film (adhesive layer) after storage, and no delamination or cracking after the bending test.
Δ: No delamination of cured film (adhesive layer) after storage, but delamination or cracking is found after the bending test.
×: Delamination of cured film (adhesive layer) is found after storage.

The results obtained in the above-mentioned tests are shown in Tables 7 (Example) and 8 (Comparative Example).

**[Table 7]**

| | Example | | | | | | |
|---|---|---|---|---|---|---|---|
| | 7 | 8 | 9 | 10 | 11 | 12 | 13 |
| transparency (haze) | ⊙ (0.05) | ⊙ (0.06) | ○ (0.8) | ⊙ (0.07) | ○ (0.6) | ⊙ (0.07) | ⊙ (0.08) |
| concave-convex surface coatability | ○ | ○ | ○ | ○ | ○ | Δ | ○ |
| adhesiveness | ⊙ | ⊙ | ○ | ⊙ | ⊙ | ○ | ○ |
| flexibility | ○ | ⊙ | ⊙ | ⊙ | ○ | ⊙ | ○ |
| water-resistant adhesiveness | ○ | ○ | ○ | ○ | ○ | ○ | ○ |

**[Table 8]**

| | Comparative Example | | | |
|---|---|---|---|---|
| | 5 | 6 | 7 | 8 |
| transparency (haze) | ○ (0.5) | ○ (0.9) | Δ (4.2) | ⊙ (0.06) |
| concave-convex surface coatability | ○ | ○ | ○ | × |
| adhesiveness | Δ | ⊙ | ○ | ○ |
| flexibility | Δ | Δ | ○ | ⊙ |
| water-resistant adhesiveness | X | Δ | ○ | ○ |

From the above-mentioned results, it can be seen that the photocurable resin compositions for adhesive (Examples 7 to 13) using di(meth)acrylate represented by the formula (1) (A-1, A-2, A-4, A-8) in the present invention are superior in transparency, coatability onto concave-convex surfaces, adhesiveness, flexibility, and water-resistant adhesiveness.

### [Industrial Applicability]

The photocurable resin composition of the present invention has low viscosity before curing, is superior in storage at low temperatures, and affords after curing a film superior in water resistance, bending resistance, and transparency.

In addition, the photocurable resin composition for adhesive of the present invention is superior in coatability onto concave-convex surfaces, and capable of forming a cured product (adhesive layer) superior in transparency, adhesiveness to uneven substrates, and flexibility during bending, and having high water-resistant adhesiveness.

This application is based on a patent application No. 2021-111779 filed in Japan and a patent application No. 2021-126067 filed in Japan, the contents of which are incorporated in full herein.

## Claims

1. A di(meth)acrylate represented by the following formula (1): 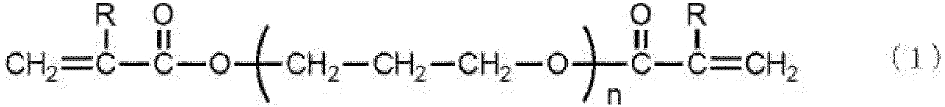 wherein R is a hydrogen atom or a methyl group, and n is an average number of moles of added oxytrimethylene group and is a real number of 5 to 90.

2. A photocurable resin composition comprising 30 to 100% by mass of component (A): di(meth)acrylate (A) according to claim 1, and 0 to 70% by mass of component (B1): urethane (meth)acrylate (B1), with respect to the total of the monomer components (100% by mass) of the composition.

3. A photocurable resin composition for adhesive, comprising
component (A): di(meth)acrylate (A) according to claim 1;
component (B): polymerizable oligomer (B) having two or more (meth)acryloyl groups in one molecule; and
component (C1): monofunctional (meth)acrylate monomer (C1), wherein
a content of component (A) is 1 to 50% by mass, a content of component (B) is 15 to 98% by mass, and a content of component (C1) is 1 to 35% by mass, with respect to a total (100% by mass) of component (A), component (B), and component (C1) .

4. The photocurable resin composition for adhesive according to claim 3, wherein the polymerizable oligomer (B) having two or more (meth)acryloyl groups in one molecule is a urethane (meth)acrylate oligomer having a polyether skeleton or a polyester skeleton.

5. A cured product obtained by curing the photocurable resin composition for adhesive according to claim 3 or 4.
